# EUROPÄISCHE PATENTANMELDUNG

(11) **EP 4 212 118 A1**
(43) Veröffentlichungstag der Anmeldung: **19.07.2023**
(21) Anmeldenummer: 22151433.4
(22) Anmeldetag: 13.01.2022
(51) Int. Cl.: A61B 18/12, A61B 90/98

(54) **INSTRUMENT UND SPEISENDES GERÄT ODER GERÄTEGRUPPE SOWIE VERFAHREN ZUM BETRIEB DERSELBEN**

(71) Anmelder: Erbe Elektromedizin GmbH, 72072 Tübingen (DE)
(72) Erfinder: LANG, Daniel, 71131 Jettingen (DE); GUTH, Andreas, 72810 Gomaringen (DE); FINK-METZ, Juergen, 73262 Reichenbach (DE)
(74) Vertreter: Rüger Abel Patentanwälte PartGmbB

(57) **Zusammenfassung**

Ein erfindungsgemäßes Instrument (11) weist Stecker (16, 17, 18) auf, mit Datenträgern (22, 29, 33), die als zusammengehörig erkennbare Codes enthalten. Solche Codes können Individualkennungen (IK1, IK2, IK3) sein, die Stecker individuell vergeben sind. Werden baugleiche Stecker typgleicher Instrumente mit Geräten oder einer Gerätegruppe verbunden, beispielsweise ein erster Stecker eines verbrauchten Instruments und ein zweiter und dritter Stecker eines neuen Instruments, welches das verbrauchte Instrument ersetzen soll, werden die drei eingesteckten Stecker als nicht zum gleichen Instrument gehörig erkannt und ein Betrieb der Geräte (12, 13, 14) und somit der Instrumente unterbunden. Damit wird die OP-Sicherheit bei Anwendung dieses Konzepts wesentlich erhöht.

## Beschreibung

Die Erfindung betrifft eine Einrichtung zur medizinischen Behandlung eines menschlichen oder tierischen Patienten. Im Weiteren betrifft die Erfindung ein Gerät oder eine Gerätegruppe zur Speisung eines solchen Instruments. Die Erfindung betrifft auch eine aus einem solchen Instrument und einen solchen Gerät oder einer Gerätegruppe bestehenden Einrichtung zur Behandlung eines menschlichen oder tierischen Patienten sowie ein Verfahren zum Betrieb dieser Einrichtung.

Zur Versorgung von chirurgischen Instrumenten mit Betriebsmedien sind medizinische Geräte in Gebrauch, die zum Beispiel hochfrequente elektrische Spannung, für den Behandlungsvorgang benötigte sonstige Medien, wie beispielsweise Gase, zum Beispiel Argon, Kryofluide oder auch Vakuum zur Absaugung von Fluiden bereitstellen.

Damit im praktischen chirurgischen Alltag sichergestellt ist, dass zueinander passende Geräte und Instrumente miteinander verbunden und Fehlverbindungen möglichst unterbunden werden, sieht die EP 2 656 806 B1 an dem Stecker eine Datenschnittstelle vor, um von dem Stecker auf das Gerät Information beispielsweise über den aktuellen Betriebszustand des Instruments, Fehlerzustände, Informationen über den Instrumententyp usw. zu übertragen. Die Datenschnittstelle ist als OneWire-Schnittstelle (Quasi-Industriestandard von Fa. Maxim Integrated) mit einem vierpoligen Kontakt ausgeführt, wobei auch andere Standards oder Kontakttypen wie UART/RS-232, SPI, I2C, USB, Mini-USB oder Mikro-SD-Schnittstelle in Betracht gezogen werden.

Außerdem ist aus der DE 10 2007 061 483 A1 ein Chirurgiegerät-Steckersystem bekannt, bei dem an dem Stecker ein Kodierstecker vorgesehen ist, der dem angeschlossenen Gerät Information übermittelt, anhand derer das Gerät gezielt Schalter zur Versorgung des Instruments mit Spannung oder Strom öffnet oder schließt.

Die insoweit vorhandenen Lösungen gehen davon aus, dass das Instrument lediglich einen einzigen mit einem Gerät zu verbindenden Stecker aufweist. Es existieren aber Instrumente mit mehreren Steckern. Werden Instrumente während der Operation gewechselt, was wegen Verschleiß oder infolge des Behandlungsverlaufs vorkommen kann, müssen alle Stecker des alten Instruments gezogen und alle Stecker des neuen Instruments korrekt wieder an ein oder mehrere entsprechende speisende Geräte angeschlossen werden. Dabei darf es nicht zu Verwechslungen kommen.

Hiervon ausgehend ist es Aufgabe der Erfindung, ein Konzept anzugeben, mit dem sich ein fehlerhafter Anschluss eines medizinischen oder chirurgischen Instruments an ein Gerät oder an mehrere Geräte mit hoher Sicherheit vermeiden lässt.

Diese Aufgabe wird unabhängig voneinander mit dem Instrument nach Anspruch 1, mit dem Gerät oder der Gerätegruppe nach Anspruch 12, der Einrichtung nach Anspruch 14, sowie dem Verfahren nach Anspruch 15 gelöst:

Das erfindungsgemäße Instrument zur medizinischen Behandlung eines menschlichen oder tierischen Patienten weist einen ersten Stecker auf, der zur Verbindung mit einem speisenden Gerät eingerichtet ist sowie mindestens einen weiteren Stecker, der ebenfalls zur Verbindung mit dem gleichen Gerät oder mit einem anderen Gerät eingerichtet ist. Beispielsweise kann das erste Gerät ein erstes Betriebsmedium, wie beispielsweise Kryofluid oder dergleichen, an das Instrument liefern. Der an dem gleichen oder einem anderen Gerät ausgebildete Steckplatz für den weiteren Stecker kann beispielsweise zur Versorgung des Instruments mit elektrischer Spannung, zum Beispiel hochfrequenter Spannung zur Gewebekoagulation oder zu anderen Zwecken, vorgesehen sein.

Unabhängig davon, wie viele Stecker an dem Instrument vorgesehen sind und unabhängig davon, ob die zugeordneten Steckplätze an einem einzigen oder an mehreren Gräten ausgebildet sind, weisen die Stecker Datenträger auf, die von dem Gerät, an dem der zugordnete Steckplatz angeordnet ist, auslesbar sind. Die Datenträger der Stecker enthalten dabei Kennungen, die so aufeinander abgestimmt sind, dass das angeschlossene Gerät oder die angeschlossenen Geräte erkennen können, ob die Stecker zu ein und demselben Instrument gehören. Ein solches Instrument ermöglicht es den Geräten zu überprüfen, ob alle Stecker, die die entsprechenden Steckplätze des Geräts oder der Geräte besetzen, zu ein und demselben Instrument gehören, d.h. als zusammengehörig anzusehen sind.

Die Überprüfung der Zusammengehörigkeit der Stecker durch die Geräte vermeidet einen sonst möglichen gravierenden Fehlerfall, der insbesondere beim Instrumentenwechsel während einer Operation auftreten kann. Wenn nämlich unter Zeitdruck Instrumente gewechselt werden sollen und ein obsoletes Instrument von den Geräten zu trennen und ein neues Instrument an die Geräte anzuschließen ist, könnte es passieren, dass ein oder zwei Stecker des obsoleten Instruments noch am jeweiligen Steckplatz verbleiben und lediglich ein oder zwei Stecker des neuen Instruments angeschlossen werden. Würde bei einem solchen Fall das vermeintlich korrekt angeschlossene neue Instrument in Betrieb genommen, kann dieses nicht ordnungsgemäß funktionieren und würde im schlimmsten Fall zu Schäden führen. Im besten Fall wäre es funktionsunfähig, was den Operationsablauf stört. Solche Ereignisse werden mit dem erfindungsgemäßen Konzept vermieden, denn es ist die Möglichkeit eröffnet, dass das speisende Gerät oder das aus mehreren Geräten bestehende Gerätesystem den Anschluss nicht zusammengehöriger Stecker, d.h. Stecker von verschiedenen Instrumenten als Fehlerfall erkennt und schnell und deutlich signalisiert.

Um eine solche Zusammengehörigkeitsprüfung zu ermöglichen, existieren mehrere Möglichkeiten. Z.B. kann der erste Stecker als Kennung Kopien der Kennungen der weiteren Stecker enthalten. Alternativ kann der erste Stecker als Kennung einen aus den Kennungen der weiteren Stecker gebildeten Rechenwert, zum Beispiel eine Prüfsumme, einen Hashcode oder dergleichen enthalten. Die Kennung eines Steckers (oder mehrerer Stecker) kann auch weitere Informationen enthalten, wie beispielsweise eine Instrumententypkennung sowie eine eigene Individualkennung.

Bei allen, insbesondere aber dem ersten Stecker kann der jeweilige Datenträger aus einem oder aus mehreren physisch gesondert ausgebildeten Einzeldatenträgern bestehen. Dies eröffnet die Möglichkeit, alle Stecker mit Datenträgern (Nur-Lese-Speichern) zu versehen, die als Individualkennung fortlaufende fest eingespeicherte Nummern tragen. Zumindest im ersten Stecker kann ein weiterer Einzeldatenträger vorgesehen sein, der einen aus den Individualkennungen gebildeten Rechenwert enthält.

Bei dem erfindungsgemäßen Instrument sind alle Stecker durch die vorhandenen Individualkennungen als zusammengehörig und somit als zum gleichen Instrument gehörig gekennzeichnet, indem in oder an dem Instrument, z.B. in einem der Stecker ein Speicherwert abrufbar ist, der sich nach einer geeigneten Bildungsvorschrift eindeutig aus den Individualkennungen der einzelnen Stecker ergibt. Die Bildungsvorschrift kann eine Prüfsummen-Bildungsvorschrift sein. Das Gerät/ die Geräte können darauf eingerichtet sein, aus den Individualkennungen nach der gleichen Bildungsvorschrift einen Rechenwert zu bilden und nur in Betrieb zu gehen, wenn der Rechenwert mit dem Speicherwert übereinstimmt.

Zur Umsetzung dieses Konzepts sind verschiedene Varianten möglich. Beispielsweise können in einer ersten Variante alle Stecker die gleiche Individualkennung aufweisen, sodass eine Gerätesteuerung lediglich prüfen muss, ob alle Individualkennungen übereinstimmen.

In einer anderen Variante enthält der erste Stecker die Individualkennungen der anderen Stecker. Damit kann die Gerätesteuerung den Betrieb des Geräts oder des Gerätesystems freigeben, sofern alle Individualkennungen, die in dem ersten Stecker gespeichert und aus diesem ausgelesen worden sind, an den Steckplätzen der anderen Stecker ausgelesen und wiedergefunden werden.

In einer dritten Variante enthält der erste Stecker einen aus den Individualkennungen der anderen Stecker gebildeten Rechenwert. Die Gerätesteuerung bildet nach der gleichen Rechenregel aus der aus den Steckplätzen der anderen Stecker ausgelesenen Individualkennung einen Rechenwert und gibt den Betrieb des Geräts oder des Gerätesystems frei, wenn dieser Rechenwert mit dem im ersten Stecker gespeicherten Rechenwert übereinstimmt.

Der Rechenwert kann auch eine Konstante sein, die für alle Instrumente gleich ist. Z.B. kann der Rechenwert die Summe der Kennungen der Stecker oder ein anderer aus den Kennungen der Stecker gebildeter Rechenwert sein. Dies eröffnet die Möglichkeit, den Rechenwert nicht in einem der Stecker, sondern nur in dem Gerät abzulegen, und zwar unabhängig von der Anzahl der zu dem System gehörigen Stecker (zwei, drei oder mehrere).

In einer weiteren Variante enthält jeder Stecker eine Individualkennung, die in die Bildung des Rechenwerts einfließt. Auch in diesem Fall geht die Gerätesteuerung nach dem vorgenannten Schema vor, wobei sie jetzt jedoch die Individualkennungen aller Stecker einbezieht.

Nach jedem der vorgenannten Prinzipien können die Kennung des ersten Steckers und die Individualkennungen der weiteren Stecker als aufeinander abgestimmte Individualkennungen angesehen werden.

Vorzugsweise weist jeder Stecker ein Kommunikationsmittel auf, über das das Gerät oder Gerätesystem an dem jeweiligen für den Stecker vorgesehenen Steckplatz mit dem Stecker in Datenaustausch gehen kann. Das Kommunikationsmittel kann ein kontaktloses Kommunikationsmodul oder ein Kontaktmodul sein. Das kontaktlose Kommunikationsmodul kann beispielsweise ein NFC-Chip oder ein optisches Modul sein. Das Kontaktmodul kann beispielsweise durch eine Kontaktgruppe gebildet sein, wie zum Beispiel die Kontakte einer USB-Schnittstelle oder dergleichen.

Das erfindungsgemäße Konzept wird auch in einem Gerät oder einer Gerätegruppe umgesetzt, mit der ein Instrument mit mehreren Steckern zu versorgen ist. Das Gerät oder die Gerätegruppe weist Steckeraufnahmen auf, die zur Aufnahme des ersten Steckers sowie des wenigstens einen weiteren Steckers eingerichtet sind. Weiter sind an den Steckeraufnahmen Kommunikationsmodule angeordnet, die darauf eingerichtet sind, mit den Kommunikationsmodulen der Stecker in Datenverbindung zu treten. Über die Kommunikationsmodule kann eine Steuereinrichtung des Geräts oder der Gerätegruppe, die Individualkennungen der Stecker auslesen und auf Konsistenz prüfen. Die Konsistenzprüfung kann nach jedem der oben genannten Prinzipien erfolgen. Bevorzugt wird eine Konsistenzprüfung auf Basis der Ermittlung eines Rechenwerts aus den Individualkennungen durchgeführt, wobei dieser Rechenwert mit einem vorzugsweise im ersten Stecker abgelegten Rechenwert übereinstimmen muss.

Die Steuereinrichtung kann von den Einzelsteuereinrichtungen der Geräte eines Gerätesystems gebildet sein, die untereinander im Datenaustausch verbunden sind, beispielsweise über einen Kommunikationsbus.

Die Steuereinrichtung ist darauf eingerichtet, die Zusammengehörigkeit der Stecker zu prüfen (Konsistenzprüfung). Dabei wird überprüft, ob die Stecker zu ein und demselben Instrument gehören. Der Betrieb des Geräts oder der Gerätegruppe wird durch die Steuereinrichtung nur dann freigegeben, wenn die Konsistenzprüfung bestanden wird, d.h. wenn die Stecker als zusammengehörig erkannt werden.

Weitere Einzelheiten vorteilhafter Ausführungsformen des erfindungsgemäßen Konzepts sind den Ansprüchen, sowie der Zeichnung und der zugehörigen Beschreibung zu entnehmen. In der Zeichnung sind Ausführungsbeispiele der Erfindung veranschaulicht. Es zeigen:
Figur 1 ein Gerätesystem mit einem angeschlossenen Instrument, in Übersichtsdarstellung,
Figur 2 bis 5 die Datenstrukturen von Steckern des Instruments nach Figur 1 in verschiedenen Ausführungsformen.

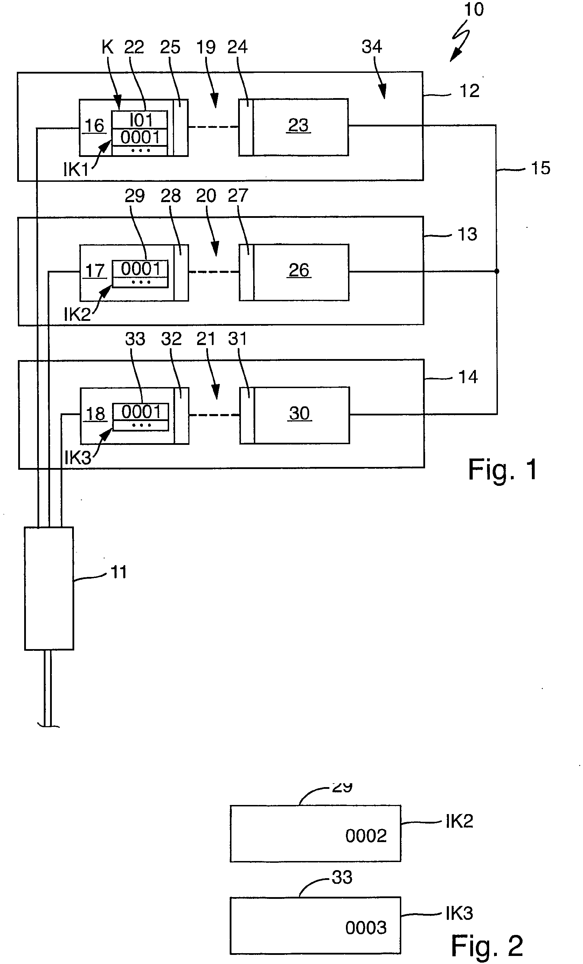

In Figur 1 ist eine Einrichtung 10 zur Behandlung eines menschlichen oder tierischen Patienten veranschaulicht. Diese Einrichtung 10 umfasst mindestens ein medizinisches oder chirurgisches Instrument 11 und ein oder mehrere Geräte 12, 13, 14, an die es angeschlossen ist. Die Geräte 12 bis 14 sind über einen Bus 15 miteinander verbunden und bilden somit ein Gerätesystem. Das Gerätesystem dient der Speisung des Instruments 11 mit den nötigen Betriebsmedien. Dazu ist das Instrument 11 mit mehreren Steckern zum Beispiel einem ersten Stecker 16 sowie wenigstens einem weiteren Stecker 17, 18 an die Geräte 12, 13, 14 angeschlossen. Die Geräte 12, 13, 14 weisen dazu entsprechende Steckplätze 19, 20, 21 auf, an denen die Stecker 16, 17, 18 die zugehörigen Betriebsmedien erhalten. Die Stecker 16, 17, 18 sind über Leitungen mit dem Instrument 11 verbunden, um dieses zu versorgen.

Der erste Stecker 16 enthält einen Datenträger 22, der aus einem oder mehreren Speicherchips bestehen kann. Auf dem Datenträger 22 ist eine Kennung K abgespeichert, die von einer Gerätesteuerung 23 auslesbar ist. Dazu ist an dem Steckplatz 19 seitens des Geräts 12 ein Kommunikationsmodul 24 vorgesehen. Der Stecker 16 beherbergt ein dazu passendes Kommunikationsmodul 25, das mit dem Kommunikationsmodul 24 in Datenverbindung treten kann. Die Datenverbindung ist zwischen beiden Kommunikationsmodulen 24, 25 in Figur 1 durch eine gestrichelte Line veranschaulicht.

In ähnlicher Weise weist das Gerät 13 eine Gerätesteuerung 26 mit einem Kommunikationsmodul 27 auf. Dieses ist dazu eingerichtet, mit einem Kommunikationsmodul 28 des Steckers 17 in Verbindung zu treten. Das Kommunikationsmodul 28 ist mit einem Datenträger 29 verbunden, auf dem eine Individualkennung IK2 abgespeichert ist. Über die Datenverbindung zwischen den Kommunikationsmodulen 27, 28 ist die Individualkennung IK2 von der Gerätesteuerung 26 lesbar.

Entsprechend weist das Gerät 14 eine Gerätesteuerung 30 auf, die über ein Kommunikationsmodul 31 mit einem Kommunikationsmodul 32 des Steckers 18 in Datenverbindung treten kann. Das Kommunikationsmodul 32 ist mit einem Datenträger 33 des Steckers 18 verbunden, auf dem eine Individualkennung IK3 abgespeichert sein kann.

Im vorliegenden extrem vereinfachten Ausführungsbeispiel besteht die Kennung K, die auf dem Datenträger 22 des ersten Steckers 16 abgespeichert ist, aus einer Instrumententypbezeichnung, hier beispielhaft I01 sowie im Weiteren aus einer Individualkennung IK1, hier die Nummer 0001. Bei den weiteren Steckern 17 und 18 ist der Datenträger 29 und 33 jeweils mit der Individualkennung IK2 und IK3 versehen, die hier im vereinfachten Beispiel ebenfalls die Nummer 0001 sind. Nach diesem Konzept tragen zusammengehörige Stecker 16, 17, 18 ein und desselben Instruments 11 übereinstimmende Individualkennungen IK1 = IK2 = IK3.

Wird ein solches Instrument 11 mit diesen Steckern 16, 17, 18 an das Gerätesystem bestehend aus den Geräten 12, 13, 14 angeschlossen, überprüft die aus den Gerätesteuerungen 23, 26, 30 bestehende Steuereinrichtung 34, ob die Individualkennungen IK1, IK2 und IK3 der Stecker 16, 17, 18 übereinstimmen. Falls ja, gibt die Gerätesteuerung 34 den Betrieb der Geräte 12, 13, 14 frei.

Figur 2 veranschaulicht ein abgewandeltes Ausführungsbeispiel, bei dem zur Vereinfachung lediglich die Datenträger 22, 29 und 33 der Stecker 16, 17, 18 veranschaulicht sind. Wiederum ist auf dem Datenspeicher 22 im Rahmen der dort niedergelegten Kennung K der Instrumententyp, hier beispielhaft I01 niedergelegt. Im Weiteren kann der Datenträger 22 die Individualkennung IK1 des ersten Steckers 16 enthalten. Der Datenträger 22 enthält die Individualkennungen IK2 und IK3, die auch in den Datenträger 29 und 30 der weiteren Stecker 17 und 18 abgespeichert sind. Bei einem solchen System prüft die Steuereinrichtung 34 vor Freigabe des Betriebs der Geräte 12 bis 14, ob an den Steckplätzen 18 und 19 tatsächlich die Kennungen IK2 und IK3 ausgelesen werden können, die in dem ersten Stecker 16 an dem Steckplatz 19 lesbar sind. Falls dies der Fall ist, ist die Steuereinrichtung darauf eingerichtet, den Betrieb freizugeben. Sie ist weiter darauf eingerichtet, den Betrieb so lange zu sperren wie dies nicht der Fall ist.

Die vorgenannten Ausführungsformen benötigen an den Steckern 16, 17, 18 Datenträger 22, 29, 33, die individuell und aufeinander abgestimmt bereitgestellt und mit den aufeinander abgestimmten Daten versehen werden. Es ist jedoch auch möglich, zumindest die Datenträger 29, 33 mit willkürlich oder zufällig festgelegten Individualkennungen IK2 und IK3 zu versehen oder derart bereitgestellte Datenträger 29, 33 zu verwenden. So können aus Serienproduktion genommene Nummerierungchips Anwendung finden, auf denen fortlaufende Nummern fest eingebrannt sind. Bei Anwendung des Ausführungsbeispiels nach Figur 2 werden dann die Individualkennungen IK2, IK3, wie sie sich beispielsweise aus laufender Produktion zufällig ergeben, auf den Datenträger 22 kopiert. Der Betrieb des Systems erfolgt nach der vorstehenden Beschreibung.

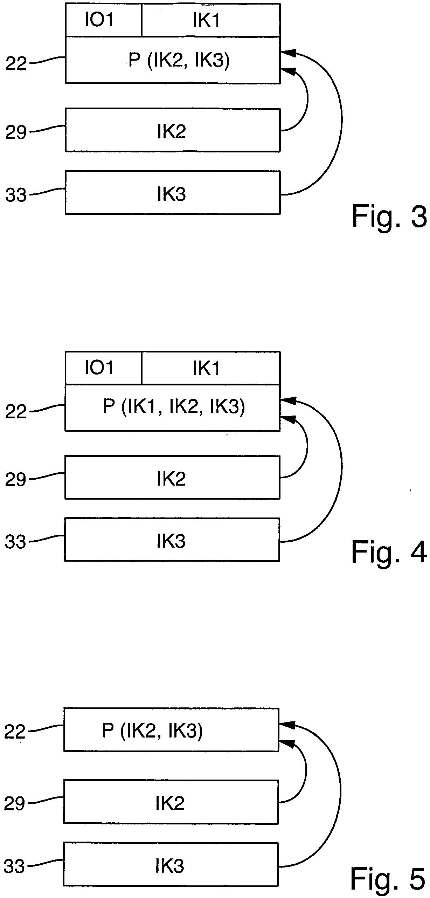

Es ist des Weiteren möglich, anstelle von Kopien der Individualkennungen IK2, IK3 lediglich einen aus diesen gebildeten Rechenwert auf dem Datenträger 22 des Steckers 16 abzuspeichern, wie es Figur 3 veranschaulicht. Der Datenträger 22 enthält dort wiederum eine Instrumentenkennung I01 und zumindest fakultativ eine eigene erste Individualkennung IK1. Die Datenträger 29, 33 enthalten ihrerseits Individualkennungen IK2, IK3, die von der Individualkennung IK1 verschieden sein können. Insbesondere können sie auch untereinander verschieden sein. Aus den beiden Individualkennungen IK2, IK3 der Stecker 17 und 18 wird beim Bereitstellen des ersten Steckers 16 ein Rechenwert P(IK2, IK3) berechnet. Dieser Rechenwert kann beispielsweise eine Prüfsumme, z.B. eine CRC-Prüfsumme, ein Hashwert oder dergleichen sein.

Nach dem Einstecken des ersten Steckers 16 prüft die Steuereinrichtung 34, ob an den weiteren Steckplätzen 20, 21 Stecker eingesteckt sind und falls ja, ob die aus dem dort eingesetzten Steckern 17 und 18 auslesbaren Individualkennungen IK2 und IK3 die Prüfsumme P(IK2, IK3) ergeben, die aus dem ersten Stecker 16 auslesbar ist. Falls ja, wird der Betrieb der Geräte 12 bis 14 freigegeben.

Weitgehend ähnlich ist die Ausführungsform nach Figur 4, bei der die Prüfsumme P(IK1, IK2, IK3) jedoch zusätzlich aus der ersten Individualkennung IK1 des ersten Steckers 16 gebildet ist. Ansonsten arbeitet das Verfahren wie vorstehend im Zusammenhang mit Figur 3 beschrieben.

Eine weitere Konfiguration ist aus Figur 5 ersichtlich. Dort enthalten die Datenträger 29, 30 der weiteren Stecker 17, 18 wieder wie bei allen vorigen Beispielen die Individualkennungen IK2 und IK3. Die auf dem Datenträger 22 des ersten Steckers 16 niedergelegte Individualkennung IK1 besteht hier jedoch lediglich aus der Prüfsumme P(IK2, IK3). Die Konsistenzprüfung, ob die drei mit den Geräten 12, 13, 14 verbundenen Stecker 16, 17, 18 zu ein und demselben Instrument 11 gehören, ist jedoch auch nach diesem vereinfachten Ausführungsbeispiel sicher möglich.

Bei allen vorbeschriebenen Ausführungsformen dient die Instrumententypkennung I01 zur Identifikation des Instrumententyps. Die Geräte 12, 13, 14 können darauf eingerichtet sein, ihre Betriebsparameter oder Bereiche für Betriebsparameter entsprechend dem Instrumententyp einzustellen. Es kann aber bei jeder hier vorgestellten Ausführungsform die Instrumententypkennung I01 auch entfallen, falls diese nicht gewünscht wird oder nicht erforderlich ist.

Ein erfindungsgemäßes Instrument 11 weist Stecker 16, 17, 18 auf, mit Datenträgern 22, 29, 33, die als zusammengehörig erkennbare Codes enthalten. Solche Codes können Individualkennungen IK1, IK2, IK3 sein, die Stecker individuell vergeben sind. Werden baugleiche Stecker typgleicher Instrumente mit Geräten oder einer Gerätegruppe verbunden, beispielsweise ein erster Stecker eines verbrauchten Instruments und ein zweiter und dritter Stecker eines neuen Instruments, welches das verbrauchte Instrument ersetzen soll, werden die drei eingesteckten Stecker als nicht zum gleichen Instrument gehörig erkannt und ein Betrieb der Geräte 12, 13, 14 und somit der Instrumente unterbunden. Damit wird die OP-Sicherheit bei Anwendung dieses Konzepts wesentlich erhöht.

### Bezugszeichen:

- 10: Einrichtung
- 11: Instrument
- 12 - 14: Geräte
- 15: Bus
- 16: erster Stecker
- 17, 18: weitere Stecker
- 19 - 21: Steckplätze
- 22: Datenträger des ersten Steckers 16
- K: Kennung
- 23: Gerätesteuerung des Geräts 12
- 24: Kommunikationsmodul des Geräts 12
- 25: Kommunikationsmodul des ersten Steckers 16
- 26: Gerätesteuerung des Geräts 13
- 27: Kommunikationsmodul des Geräts 13
- 28: Kommunikationsmodul des weiteren Steckers 17
- 29: Datenträger des weiteren Steckers 17
- 30: Gerätesteuerung des Geräts 14
- 31: Kommunikationsmodul des Geräts 14
- 32: Kommunikationsmodul des weiteren Steckers 18
- 33: Datenträger des weiteren Steckers 18
- 34: Steuereinrichtung

## Patentansprüche

1. Instrument (11) zur medizinischen Behandlung eines menschlichen oder tierischen Patienten,
mit einem ersten Stecker (16), der zur Verbindung mit einem Gerät (12) eingerichtet ist,
mit mindestens einem weiteren Stecker (17, 18), der zur Verbindung mit dem Gerät (12) oder einem anderen Gerät (13, 14) eingerichtet ist,
wobei der erste Stecker (16) einen Datenträger (22) mit einer Kennung (K) enthält und
wobei der weitere Stecker (17, 18) einen Datenträger (29, 33) mit einer weiteren Individualkennung (IK2, IK3) enthält.

2. Instrument nach Anspruch 1, **dadurch gekennzeichnet, dass** die erste Kennung (K) eine Instrumentenkennung (I) sowie eine Individualkennung (IK1) enthält.

3. Instrument nach Anspruch 1, **dadurch gekennzeichnet, dass** alle Stecker (16, 17, 18) Datenträger (22, 29, 33) mit aufeinander abgestimmten Individualkennungen (IK1, IK2, IK3) aufweisen.

4. Instrument nach Anspruch 1, **dadurch gekennzeichnet, dass** der Datenträger (22) des ersten Steckers (16) die Individualkennungen (IK2, IK3) der anderen Stecker (17, 18) oder eine darauf basierende Rechengröße aufweist.

5. Instrument nach Anspruch 1, **dadurch gekennzeichnet, dass** der Datenträger (22) des ersten Steckers (16) einen Hashcode oder eine Prüfkennung (P) der Individualkennungen (IK2, IK3) der anderen Stecker (17, 18) aufweist.

6. Instrument nach Anspruch 1, **dadurch gekennzeichnet, dass** der Datenträger (22) des ersten Steckers (16) einen Hashcode oder eine Prüfkennung (P) der Individualkennungen (IK1, IK2, IK3) aller Stecker (16, 17, 18) aufweist.

7. Instrument nach einem der vorstehenden Ansprüche, **dadurch gekennzeichnet, dass** die Stecker (16, 17, 18) jeweils mit einem Kommunikationsmittel (25, 28, 32) verbunden sind, über das wenigstens die Individualkennung (IK1, IK2, IK3) an ein anzuschließendes Gerät übertragbar ist.

8. Instrument nach Anspruch 7, **dadurch gekennzeichnet, dass** das Kommunikationsmittel (25, 28, 32) ein Funkmodul ist.

9. Instrument nach Anspruch 7, **dadurch gekennzeichnet, dass** das Kommunikationsmittel (25, 28, 32) ein Kontaktmodul ist.

10. Instrument nach einem der vorstehenden Ansprüche, **dadurch gekennzeichnet, dass** der erste Stecker (16) und der mindestens eine weitere Stecker (17, 18) zur Übertragung unterschiedlicher Medien eingerichtet sind.

11. Gerät oder Gerätegruppe (12, 13, 14) zur Speisung eines Instruments (11) nach einem der vorstehenden Ansprüche,
mit Steckeraufnahmen (19, 20, 21), die zur Aufnahme eines ersten Steckers (16) und wenigstens eines weiteren Steckers (17, 18) sowie zur Erfassung deren Individualkennungen (IK1, IK2, IK3) eingerichtet sind,
mit einer Steuereinrichtung (34), die darauf eingerichtet ist, die Zusammengehörigkeit der Stecker (16, 17, 18) zu prüfen.

12. Gerät oder Gerätegruppe (12, 13, 14) nach Anspruch 11, **dadurch gekennzeichnet, dass** die Steuereinrichtung (34) darauf eingerichtet ist, die Zusammengehörigkeit der Stecker (16, 17, 18) auf Basis der Individualkennungen (IK1, IK2, IK3) der Stecker (16, 17, 18) zu überprüfen.

13. Gerät oder Gerätegruppe (12, 13, 14) nach einem der Ansprüche 10 oder 11, **dadurch gekennzeichnet, dass** die Steuereinrichtung (34) darauf eingerichtet ist, den Betrieb des Instruments (11) nur freizugeben, wenn die mit dem Gerät (12) oder den Geräten (12, 13, 14) verbundenen Stecker (16, 17, 18) des Instruments (11) als zusammengehörig erkannt sind.

14. Einrichtung zur Behandlung eines menschlichen oder tierischen Patienten, bestehend aus einem Instrument (11) nach einem der Ansprüche 1 bis 9 und einem Gerät oder einer Gerätegruppe (12, 13, 14) nach einem der Ansprüche 10 oder 11.

15. Verfahren zum Betrieb einer Einrichtung nach Anspruch 14, **dadurch gekennzeichnet, dass** nach dem Verbinden der Stecker (16, 17, 18) mit den Steckeraufnahmen (19, 20, 21) auf Basis in den Steckern (16, 17, 18) abgespeicherten Kennungen geprüft wird, ob die Stecker (16, 17, 18) demselben Instrument (11) gehören, und der Betrieb des Instruments (11) nur freigegeben wird, wenn die Stecker (16, 17, 18) zu demselben Instrument (11) gehören.
